(11) **EP 1 863 411 B1**

(12) **EUROPÄISCHE PATENTSCHRIFT**

(45) Veröffentlichungstag und Bekanntmachung des Hinweises auf die Patenterteilung:
**13.08.2014 Patentblatt 2014/33**

(21) Anmeldenummer: **05802062.9**

(22) Anmeldetag: **26.10.2005**

(51) Int Cl.:
***A61F 2/16*** *(2006.01)*

(86) Internationale Anmeldenummer:
**PCT/EP2005/011473**

(87) Internationale Veröffentlichungsnummer:
**WO 2006/099893 (28.09.2006 Gazette 2006/39)**

(54) **Intraokularlinse**

Intraocular lens

Lentille intra-oculaire

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **24.03.2005 DE 102005013877**
**20.05.2005 DE 102005023480**

(43) Veröffentlichungstag der Anmeldung:
**12.12.2007 Patentblatt 2007/50**

(73) Patentinhaber: **Carl Zeiss Meditec AG**
**07745 Jena (DE)**

(72) Erfinder:
• **STORK, Wilhelm**
**76831 Impflingen (DE)**
• **PETERSEN, Jörgen**
**37077 Göttingen (DE)**

(74) Vertreter: **Eisenführ Speiser**
**Patentanwälte Rechtsanwälte PartGmbB**
**Postfach 31 02 60**
**80102 München (DE)**

(56) Entgegenhaltungen:
**EP-A- 0 391 452      WO-A-03/007849**
**US-A- 6 015 435      US-B1- 6 666 887**

**Beschreibung**

Die Erfindung betrifft eine Intraokularlinse. Eine solche Linse ist beispielsweise aus der EP 1 185 220 B1 derselben Inhaberin bekannt.

Intraokularlinsen im Allgemeinen setzen sich zusammen aus einem optischen Linsenteil und einem sich peripher anschließenden haptischen Linsenteil zur Fixierung der Intraokularlinse im Auge. Die Abbildungseigenschaften des optischen Linsenteils sind auf die Verhältnisse des zu behandelnden Auges eingestellt. Dabei können sowohl Linsen mit rein refraktiven Eigenschaften als auch solche mit überlagerten diffraktiven Eigenschaften zum Einsatz kommen. Beispielsweise wird in der EP 1 185 220 B1 eine Linse vorgestellt, die in einem zentralen Linsenbereich die Lichtstrahlen zum Einen refraktiv fokussiert, wobei demselben Linsenbereich eine diffraktive Feinstruktur überlagert ist, die wenigstens einen zweiten Fokus erzeugt. Mit einer solchen Linse kann der Patient sowohl in der Ferne als auch in der Nähe scharf sehen.

Ferner ist es aus WO 03/007 849 A bekannt, solche Linsen in einem Randbereich in konzentrische ringförmige Zonen zu unterteilen, die in Richtung der optischen Achse so versetzt sind, dass der Weglängenunterschied des Strahlengangs im Übergang von jeweils benachbarten Zonen ein ganzzahliges Vielfaches der Designwellenlänge der Linse ist. Diese Linsengestaltung bewirkt dass eine Linse unter Beibehaltung ihrer Brechkraft, welche von der Linsenrückfläche mit bekannt wird, und bei gleicher optischer Fläche wesentlich dünner ausgestaltet werden kann. Eine solche Linse ist für minimal invasive Eingriffe am Auge besonders geeignet, da sie auch bei größeren Brechkräften gut zusammenfaltbar ist.

Insbesondere im Zusammenhang mit einer Vitrektomie, bei der der Glaskörper im Augeninneren entfernt wird, stellt sich aber das Problem, dass sich in der Regel die Abbildungseigenschaften der davor liegenden Linse ändern. Dies hängt mit den unterschiedlichen Brechungsindizes der bei dieser Operation eingefüllten Tamponade bzw. des Substitutionsmediums für den Glaskörper zusammen. Bekannt sind beispielsweise Wasser-, Gas- oder Silikonöl-Einfüllungen. Auch Mischsubstanzen auf Basis teil-flourierter Alkane und Öl kommen zur Anwendung, die jeweils unterschiedliche Brechungsindizes haben.

Aufgabe der Erfindung ist es daher, eine Intraokularlinse zu schaffen, bei der Abbildungsfehler unabhängig von dem eingefüllten Substitutionsmedium so gering wie möglich sind.

Diese Aufgabe wird durch eine Intraokularlinse nach Patentanspruch 1 gelöst.

Die erfindungsgemäße Intraokularlinse weist im Bereich Ihres optischen Linsenteils entlang der der Retina zugewandten Rückfläche wenigstens eine optisch wirksame Teilfläche mit einem konkaven, zumindest näherungsweise sphärischen Krümmungsverlauf auf, dessen Krümmungsradius dem Abstand der Teilfläche zur Retina im Bereich größter Sehschärfe entspricht.

Die Intraokularlinse kann an verschiedenen Stellen im Auge implantiert werden, beispielsweise als Vorderkammerlinse, d. h. zwischen Hornhaut und Iris, oder als Hinterkammerlinse, d. h. hinter der Iris und vor dem Glaskörper, meist in den Kapselsack der natürlichen Linse. Ferner variiert der Abstand zur Retina abhängig von der Größe und Form des Augapfels. Hierauf sind die Abbildungseigenschaften der Linse unter Berücksichtigung des optischen Gesamtsystems, Cornea, evtl Vorderkammerlinse und Intraokularlinse eingestellt und entsprechend wird auch die Krümmung der Rückfläche auf die Abbildungseigenschaften der Vorderfläche angepasst.

Die Krümmungen der Teilflächen sind dabei erfindungsgemäß zumindest näherungsweise sphärisch, d.h. im Idealfall tatsächlich sphärisch oder zum Beispiel parabolisch, ausgebildet, so dass die Abweichung von der Kugelflächenform zumindest in der Nähe des Zentrums der Teilfläche vernachlässigbar ist. Dadurch, dass der Krümmungsmittelpunkt der konkaven Teilflächen auf der Retina und zwar im Bereich größter Sehschärfe, d.h. in der Makula oder der Fovea, liegt, wird bewirkt, dass die nach Brechung und/oder Beugung an der Vorderfläche auf die Rückfläche einfallende Wellenfront dort unabhängig von den Brechungsindizes der Linse und des dahinter befindlichen Mediums nicht zusätzlich gebrochen wird.

Anders gesagt wird bei einer so eingestellten erfindungsgemäßen Linse parallel zur optischen Achse einfallendes Licht ausschließlich bei Eintritt durch deren Vorderfläche in ein auf die Retina fokussiertes Strahlenbündel gebrochen und/oder gebeugt. Dieses Strahlenbündel durchläuft die Rückfläche ohne nochmalige Brechung, da es stets senkrecht auf der Rückfläche der Linse steht. Hierdurch wird sichergestellt, dass der geometrische Abstand zwischen der Intraokularlinse und dem Brennpunkt des optischen Gesamtsystems unabhängig von der Tamponade oder dem Substitutionsmedium immer gleich bleibt.

Kleinere Abweichungen von der Sphärizität erzeugen dabei nur eine geringe Brechung an der Rückfläche und damit kleinere Abbildungsfehler durch die Verschiebung des Brennpunkts. Eine leichte Asphärizität kann sogar zur Korrektur von Abbildungsfehlern der Vorderfläche und des Gesamtsystems vorgesehen werden. Es ist bei der Gestaltung der Rückfläche aber darauf zu achten, dass deren Krümmung so weit an die Sphärenform genähert wird, dass unabhängig von dem Brechungsindex der verwendeten Tamponade bzw. des Glaskörpers, die Abbildungsfehler durch die Brechung am Übergang von der Linsenrückfläche zum Glaskörpervolumen von dem Patienten nicht störend bemerkt werden.

In einer vorteilhaften Ausgestaltung beträgt der Krümmungsradius zwischen 12 mm und 30 mm und besonders bevorzugt zwischen 13 mm und 23 mm. Dieser Bereich deckt die meisten erfahrungsgemäß auftretenden Abstände der Intraokularlinse von der Retina, abhängig von dem Implantationsort und der Größe und Form des Augapfels, ab.

Bevorzugt ist eine erste optisch wirksame Teilfläche mit zumindest näherungsweise sphärischem Krümmungsverlauf in einem zentralen, rein refraktiven Linsenbereich der Rückfläche ausgebildet. Diese Teilfläche kann nur einen Teil oder die gesamte optisch wirksame Rückfläche überdecken.

Vorteilhaft ist es ferner wenn wenigstens eine zweite optisch wirksame Teilfläche mit zumindest näherungsweise sphärischem Krümmungsverlauf in einem den zentralen Linsenbereich umgebenden diffraktiven Linsenbereich der Rückfläche konzentrisch an der ersten optisch wirksamen Teilfläche angrenzend ausgebildet ist, wobei der Krümmungsradius der zweiten optisch wirksamen Teilfläche deren Abstand zur Retina im Bereich größter Sehschärfe entspricht. Bevorzugt ist dabei der Weglängenunterschied des Strahlenganges im Übergang von der ersten optisch wirksamen Teilfläche zur zweiten optisch wirksamen Teilfläche ein ganzzahliges Vielfaches von n ≥1 der Designwellenlänge.

An die zweite optisch wirksame Teilfläche können sich in einer vorteilhaften Ausgestaltung weitere optisch wirksame Teilflächen mit zumindest näherungsweise sphärischem Krümmungsverlauf in dem ringförmigen diffraktiven Linsenbereich konzentrisch an die zweiten optisch wirksamen Teilfläche und des weiteren aneinander angrenzend anschließen, wobei der Krümmungsradius der weiteren optisch wirksamen Teilflächen jeweils deren Abstand zur Retina im Bereich größter Sehschärfe entspricht. Bevorzugt ist auch hier der Weglängenunterschied des Strahlenganges im Übergang von den jeweils benachbarten optisch wirksamen Teilflächen ein ganzzahliges Vielfaches von n ≥1 der Designwellenlänge.

Bei diesen Ausführungsformen ist die Rückfläche in mehrere Teilflächen unterteilt, die ringförmige konzentrische Zonen bilden. Diese Linsengestaltung bewirkt, dass eine Linse unter Beibehaltung der optischen Eigenschaften, insbesondere des geometrischen Abstands zwischen der Intraokularlinse und dem Brennpunkt des optischen Gesamtsystems unabhängig von der Tamponade oder dem Substitutionsmedium, bei gleicher optischer Fläche wesentlich dünner ausgestaltet werden kann und deshalb für minimal invasive Eingriffe am Auge besonders geeignet ist.

Die Beibehaltung der Weglängenunterschiede zwischen jeweils zwei benachbarten Teilflächen oder Zonen wird in einer bevorzugten Ausführungsform durch eine geometrische Stufenhöhe $h$ parallel zum Strahlverlauf, d.h. der Differenz der Krümmungsradien zweier benachbarter Teilflächen/Zonen, erreicht, die der folgenden Gleichung genügt

$$h \cdot (n_{IOL} - n_{Tamponade1}) \cdot N = h \cdot (n_{IOL} - n_{Tamponade2}) \cdot M \, ,$$

wobei $N$ und $M$ ganze Zahlen sind und $n_{IOL}$, $n_{Tamponade1}$ bzw. $n_{Tamponade2}$ die Brechungsindices der Intraokularlinse und zweier unterschiedlicher bestimmter Tamponaden im Glaskörper des Auges sind.

Im Allgemeinen kann die Rückfläche also rein refraktiv oder gemischt refraktivdiffraktiv gestaltet sein. Hierbei wird unter refraktiv eine näherungsweise sphärische (Teil-)Fläche verstanden, die zumindest für achsparallel einfallende Strahlen im Idealfall gerade keine zusätzliche Brechung erzeugen. Für Strahlen, die unter einem Winkel zur optischen Achse einfallen werden zwar dennoch auch an der Rückfläche gebrochen, jedoch zumindest geringfügiger als bei bekannten Intraokularlinsen und im Wesentlichen nur mit dem Effekt einer Veränderung der Größe der Abbildung bei unterschiedlichen Tamponaden oder Substitutionsmedien. Es kommt dabei darauf an, dass in der Summe der optischen Eigenschaften der Rückfläche (refraktiv und/oder diffraktiv) diese, zumindest für das axiale Strahlenbündel, keine Veränderung der Wellenfront bewirkt.

Bevorzugt sind die optisch wirksamen Teilflächen sägezahnförmig ausgebildet.

Bevorzugt weist der zentrale Linsenbereich einen Durchmesser von etwa 4 mm auf.

Weiterhin bevorzugt weist der optische Linsenteil insgesamt, d.h. auch auf der Vorderfläche einen zentralen Linsenbereich und einen diesen umgebenden ringförmigen Linsenbereich mit einem gemeinsamen Fokus auf. Der ringförmige Linsenbereich weist dabei wenigstens eine erste konzentrische ringförmige Zone auf, wobei der Weglängenunterschied des Strahlengangs dem Übergang vom zentralen Linsenbereich zur ersten ringförmigen Zone und im Übergang weiterer, jeweils benachbarter ringförmiger Zonen ein ganzzahliges Vielfaches von n ≥1 der Designwellenlänge ist.

Bei einer vorteilhaften Weiterbildung erstreckt sich über die Vorderfläche zumindest abschnittsweise eine diffraktive Feinstruktur zur Bildung einer multifokalen, insbesondere bifokalen, Linse. In aller Regel reicht es aus, wenn sich die diffraktive Feinstruktur nur über den zentralen Linsenbereich erstreckt, da insbesondere die bifokale Funktion nur bei einer dem Tageslicht entsprechenden Helligkeit erforderlich ist, und die Pupillenöffnung des Auges im Wesentlichen nur im Bereich des zentralen Linsenbereichs geöffnet ist. Die zusätzliche diffraktive Feinstruktur kann insbesondere in Form von um die optische Linsenachse angeordneten konzentrischen Zonen oder (Unter-) Teilflächen ausgebildet sein. Der Weglängenunterschied des Strahlenganges im Übergang von jeweils benachbarten Zonen oder (Unter-)Teilflächen der diffraktiven Feinstruktur auf der Vorderfläche beträgt im Fall einer bifokalen Linse ein Bruchteil der Designwellenlänge, bevorzugt abwechselnd das 0,5 bis 0,8-fache bzw. das 0,5 bis 0,2-fache und besonders bevorzugt das 0,4- bzw. 0,6-fache der Designwellenlänge.

Durch die konkave Rückfläche der Intraokularlinse muss die Vorderfläche eine höhere Brechkraft und damit Krümmung aufweisen. Hierdurch erhält die Linse eine größerer Mittendicke als eine Bikonvexe Linse. Um dem entgegenzuwirken ist die Linse vorteilhafterweise als diffraktiv-refraktive Linse ausgestaltet. Weil so bei gleicher Brechkraft eine geringerer Mittedicke erreicht wird, kombiniert diese bevorzugte Ausführungsform die vorteilhaften Abbildungseigenschaften der erfindungsgemäßen Intraokularlinse mit dem Vorteil eines flachen Designs, so dass sie sich zum Zweck der Implantation falten lässt und somit einen minimal invasiven Eingriff ermöglicht. Der Weglängenunterschied der benachbarten Zonen kann zum Einen durch den Brechungsindex bzw. durch eine entsprechende Materialwahl und/oder zum Anderen durch die Geometrie der jeweiligen Zone eingestellt werden. Die ringförmigen Zonen sind im Querschnitt dabei vorteilhafter Weise sägezahnartig ausgebildet.

Weitere Merkmale und Vorteile der Erfindung werden nun anhand von Ausführungsbeispielen mit Hilfe der Zeichnungen näher erläutert: Es zeigen:

Fig. 1    eine schematische Darstellung des Strahlenverlaufs durch das Auge mit erfindungsgemäßer Intraokularlinse;

Fig. 2    ein Schnittbild durch eine Hälfte eines Linsenkörpers der erfindungsgemäßen Intraokularlinse;

Fig. 3    eine vergrößerte Darstellung eines Abschnitts der Linsenfront zur Erläuterung einer zusätzlichen diffraktiven Feinstruktur;

Fig. 4    ein Schnittbild durch eine Hälfte eines weiteren Ausführungsbeispiels eines erfindungsgemäßen Linsenkörpers;

Fig. 5    ein Schnittbild durch ein weiteres Ausführungsbeispiel der erfindungsgemäßen Intraokularlinse;

Fig. 6    ein Schnittbild durch eine Hälfte eines weiteren Beispiels eines Linsenkörpers;

Fig. 7    erfindungsgemäße Linse mit rein refraktiver Rückfläche; und

Fig.8    eine vergrößerte Darstellung eines Abschnitts der Linsenfront zur Erläuterung einer zusätzlichen diffraktiven Feinstruktur.

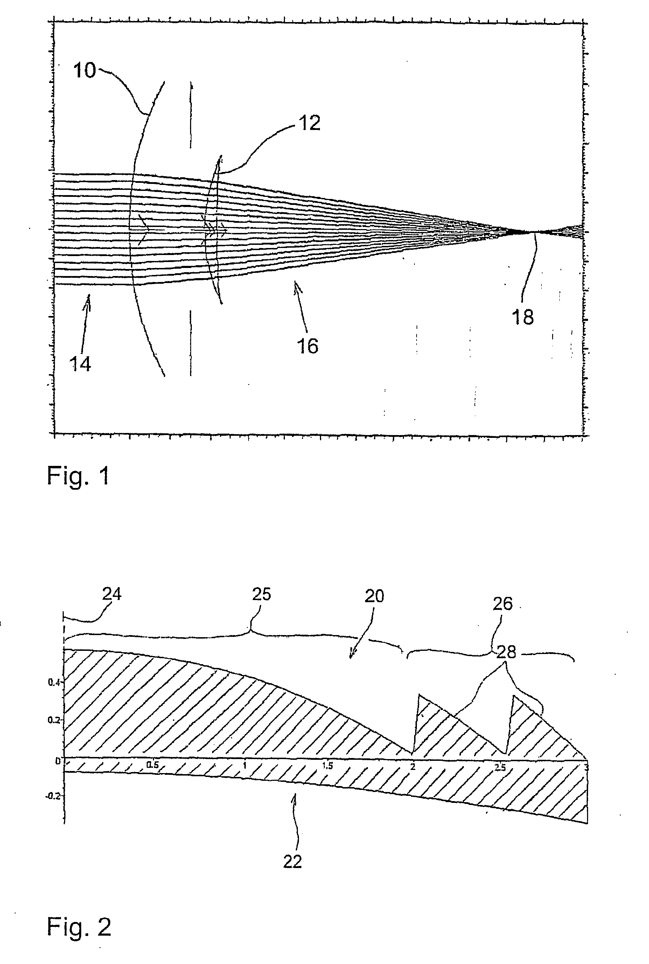

Die im Folgenden beschriebenen Abbildungsverhältnisse in Fig. 1 sind aus einem mittels eines Optik-Design Tools erstellten Simulationsmodell entstanden. Fig. 1 zeigt einen schematisierten Ausschnitt eines Auges mit einer ebenfalls schematisierten Darstellung der erfindungsgemäßen Intraokularlinse 12. Ein von einem unendlich weit entfernten Gegenstand ausgehendes achparalleles Strahlenbündel 14 passiert zunächst die Cornea des Auges, die hier schematisch als eine wirksame Fläche 10 dargestellt ist, und die Vorderkammer des Auges und wird bereits auf seinem Weg zur Intraokularlinse aufgrund unterschiedlicher Brechungsindizes gebrochen. Beim Erreichen der Intraokularlinse wird das achsparallele Strahlenbündel daher bereits in einen Eingangsstrahlenbündel mit gekrümmter Wellenfront gebrochen. Diese Wellenfront hat in der Regel eine näherungsweise sphärischer Krümmung, kann aber auch stark asphärisch gekrümmt sein. Dieser Umstand kann entsprechend bei dem Design der Intraokularlinse 12, d. h. bei der Berechnung deren Abbildungseigenschaften und somit der Krümmung der Vorderfläche, unter anderem in Abhängigkeit von deren Brechungsindex, berücksichtigt werden. Das auf die Vorderfläche der Intraokularlinse 12 auftreffende Strahlenbündel 14 wird aufgrund deren Abbildungseigenschaften in ein ausgehendes, auf die Retina fokussiertes Strahlenbündel 16 gebrochen und/oder gebeugt, je nach dem, ob es sich um eine Linse mit refraktivem, diffraktivem (Fresnel-) oder gemischt refraktivdiffraktivem Anteil handelt. Die Rückfläche der Intraokularlinse weist erfindungsgemäß einen sphärischen Krümmungsverlauf auf dessen Radius allein von dem Abstand der implantierten Intraokularlinse zur Retina im Fokuspunkt 18, also auf der optischen Achse oder zentralen Linsenachse, abhängt. Dadurch entspricht die Krümmung der Rückfläche der Krümmung der Wellenfronten des auslaufenden Strahlenbündels 16, das dort nicht nochmals gebeugt und/oder gebrochen wird.

Der in Fig. 2 dargestellte Halbschnitt einer erfindungsgemäßen Intraokularlinse zeigt deren positiv refraktive Vorderfläche 20, die in einen zentralen Linsenbereich 25 mit rein refraktiver Eigenschaft und einen diesen konzentrisch umgebenden ringförmigen Linsenbereich 26 mit überlagerten positiv diffraktiven, ringförmigen Zonen 28 unterteilt ist. Die Rückfläche 22 weist durchgehend einen sphärischen Krümmungsverlauf auf während die Vorderfläche 20 insgesamt einen asphärischen Krümmungsverlauf aufweist. Die Oberflächen der in dem ringförmigen Linsenbereich 26 vorgesehenen zwei konzentrischen ringförmigen Zonen 28 sind jeweils dergestalt in Richtung der optischen Achse gegeneinander sägezahnartig versetzt, dass im Übergang von dem zentralen Linsenbereich 25 zu der inneren ringförmigen Zone sowie im Übergang von der inneren zur äußeren ringförmigen Zone der Weglängenunterschied des Strahlengangs ein ganzzahliges Vielfaches von der Designwellenlänge beträgt. Die Designwellenlänge beträgt üblicherweise 550 nm, liegt also im Bereich grünen Lichts. Durch diese Maßnahme kann die Dicke der Intraokularlinse verringert werden, so dass diese faltbar und somit leichter implantierbar ist, ohne die Abbildungseigenschaften wesentlich zu ändern. Der zentrale Linsenbereich hat in dem gezeigten Beispiel etwa einen Durchmesser von 4 mm, während der sich im Randbereich des optischen Linsenteils befindende ringförmige Linsenbereich 26 eine Breite von in diesem Fall 1 mm aufweist.

Dadurch, dass aufgrund der sphärischen Form der Rückfläche 22 die Brechung der Intraokularlinse für achsparallele Strahlen entlang dieser Fläche 0 ist, verändert sich die Lage des Fokus für diese Strahlen in Abhängigkeit von dem Brechungsindex des hinter der Intraokularlinse liegenden Mediums nicht. Lediglich der Abbildungsmaßstab kann sich um den folgenden Wert verändern

$$m = \frac{n_{Tamponade}}{n_{Glaskörper}}.$$

Wird beispielsweise der Glaskörper des Auges nach einer Vitrektomie zunächst durch Silikonöl ($n_{Silikonöl}$ = 1,43) ersetzt und später das Silikonöl gegen Wasser ausgetauscht (n = 1,336), hat dies zur Folge, dass der Patient ein um 7 % vergrößertes Bild sieht.

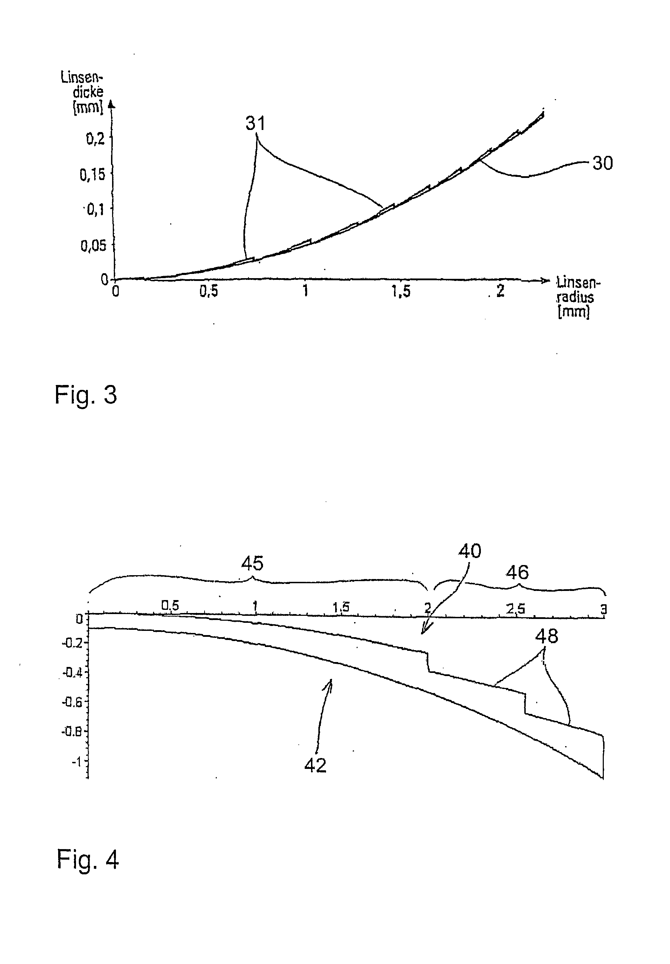

Zusätzlich zu dem refraktiven Anteil des die Linse passierenden Lichts, welcher sowohl im zentralen Linsenbereich 25 als auch im peripheren ringförmigen Linsenbereich 26 gebildet wird, kann bei einer Intraokularlinse gemäß Fig. 3 mittels einer diffraktiven Feinstruktur 31, hier nur auf der Vorderfläche 30 der Intraokularlinse, ein bestimmbarer Anteil des Lichts gebeugt werden. Während der Fokus des refraktiven Anteils beispielsweise für das Sehen im Fernbereich eingestellt ist, kann durch ein auf die Verhältnisse im Auge des Patienten abgestimmtes Design der diffraktiven Feinstruktur 31 ein zweiter Fokus gebildet werden, der das scharfe Sehen im Nahbereich ermöglicht. In diesem Fall spricht man von einer bifokalen Linse. Die Feinstruktur ist bevorzugt als diffraktives Muster ausgebildet und besitzt die Form ringförmiger Feinstrukturelemente, die in der Regel konzentrisch zur optischen Achse und sägezahnförmig in Richtung der optischen Achse versetzt ausgebildet sind.

Die Fig. 3 zeigt den im Wesentlichen sphärischen Anteil der Schnittkurve der Vorderfläche im zentralen Linsenbereich. Ausgehend von der im Wesentlichen sphärischen Grundkurve 30 besitzen die ringförmigen diffraktiven Zonen Tiefen von 1,0 $\mu$m bis 5,0 $\mu$m. Diese Tiefen der diffraktiven Feintrukturen bifokaler Linsen ergeben einen Weglängenunterschied zwischen benachbarten Zonen eines Bruchteils, z. B. 0,4 bzw. 0,6, der Designwellenlänge, typischerweise wiederum grünes Licht bei einer Wellenlänge von 550 nm.

Das zusätzliche diffraktive Feinstrukturmuster ist bevorzugt im zentralen Linsenbereich vorgesehen. Es kann sich jedoch auch über den ringförmigen Linsenbereich 26 erstrecken und die in diesem Bereich befindlichen ringförmigen Zonen überlagern. Ein weiteres Ausführungsbeispiel einer erfindungsgemäßen Intraokularlinse ist in Fig. 4 wiederum als Halbschnitt dargestellt. Hierbei handelt es sich um eine Linse mit positiv refraktiver (konvexer) Frontfläche 40, die zusätzlich zu dem zentralen Linsenbereich 45 mit rein refraktiver Eigenschaft einen diesen konzentrisch umgebenden ringförmigen Randbereich 46 mit überlagerten negativ diffraktiven, ringförmigen Zonen 48 unterteilt ist. Die Rückfläche 42 weist wiederum durchgehend einen sphärischen Krümmungsverlauf auf. Bei dieser Linse ist die effektive lokale Krümmung der Frontfläche sowohl in dem Zentralen Linsenbereich 45 als auch in den einzelnen ringförmigen Zonen 48 kleiner als die makroskopische Krümmung der gesamten Linsenfront.

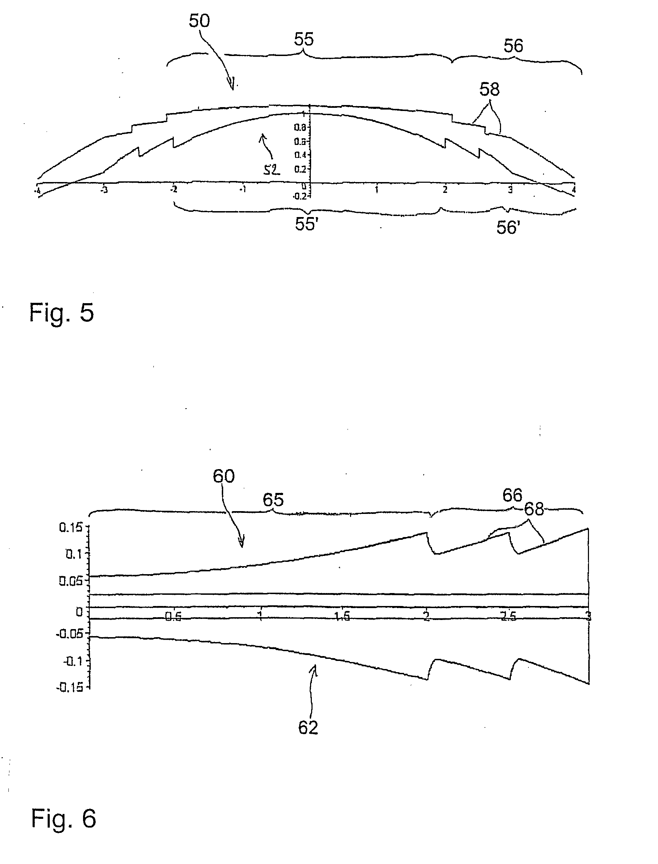

Fig. 5 zeigt noch ein Ausführungsbeispiel einer erfindungsgemäßen Intraokularlinse. Auch in diesem Beispiel ist die Frontfläche 50 positiv refraktiv (konvex) mit einem rein refraktiven zentralen Linsenbereich 55 und einem Randbereich 56 mit überlagerten negativ diffraktiven, konzentrisch ringförmigen Zonen 58. Die konkave Rückfläche 52 ist hier aber ihrerseits in einen zentralen, rein refraktiven Bereich 55' und einen ringförmigen Randbereich 56' mit überlagerten diffraktiven Strukturen 58' sägezahnförmiger konzentrischer Ringe unterteilt. Dadurch ist die effektive lokale Krümmung der Rückfläche 52 in dem zentralen Linsenbereich 55' als auch in den einzelnen ringförmigen Zonen 56' größer als deren makroskopische Krümmung. Insgesamt wird somit eine sehr dünne Linse erzeugt.

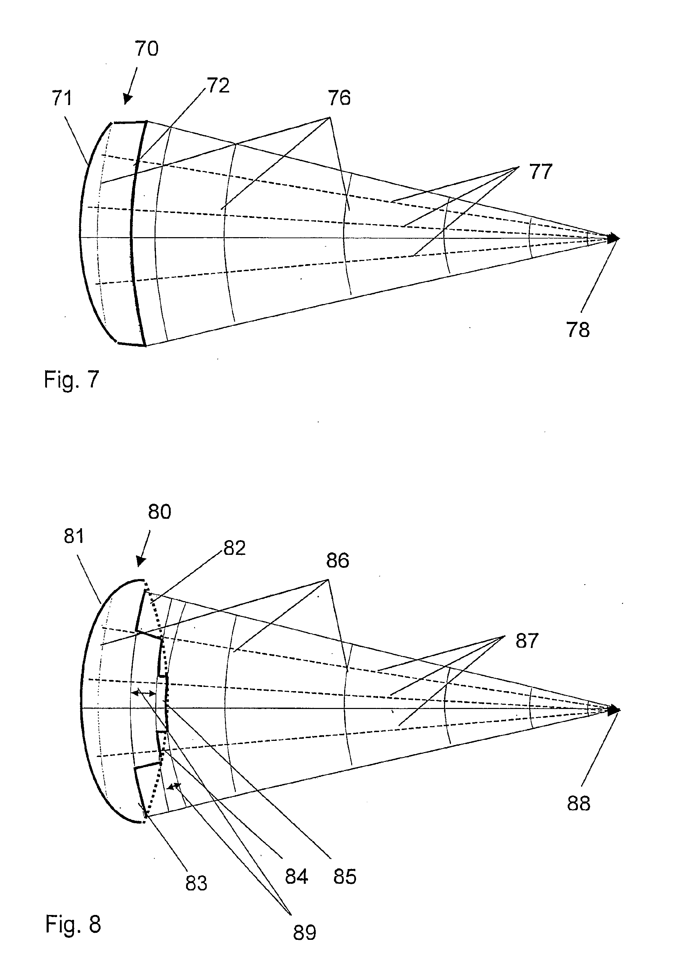

Das Beispiel gemäß Fig. 6, nur im Halbschnitt dargestellt, weist erstmals eine konkave Frontfläche 60 mit einem Randbereich 66 mit überlagerter negativ diffraktiven, konzentrisch ringförmigen Zonen 68 auf. Hier ist die effektive lokale Krümmung der Frontfläche 60 sowohl in dem zentralen Linsenbereich 65 als auch in den einzelnen ringförmigen Zonen 68 größer als die makroskopische Krümmung der gesamten Linsenfront. Die konvexe Rückfläche 62 ist wie in dem vorangegangenen Ausführungsbeispiel gemäß Fig. 5 ausgebildet, wodurch insgesamt ebenfalls eine verhältnismäßig dünnes Linsendesign erreicht wird. Das Ausführungsbeispiel der erfindungsgemäßen Intraokularlinse (70) gemäß Fig. 7 weist rein refraktive Grenzflächen, d.h. eine Vorderfläche (71) und eine Rückfläche (72) ohne diffraktive Strukturen, auf. Eine brechende Wirkung wird jedoch nur von der der Retina abgewandten Vorderfläche (71) erzeugt. Die Krümmung der Rückfläche entspricht der Krümmung der Wellenfronten (76) am rückwärtigen Linsenrand also am Übergang von der Linse zum Glaskörpermedium. Die auf den Wellenfronten senkrecht stehenden Strahlen (77) treffen unter einem rechten Winkel auf die Rückfläche und werden dem gemäß nicht gebrochen. Der Abstand des Fokus (78) zur Intraokularlinse und damit zu dem optischen Gesamtsystem bleibt daher unabhängig von dem Brechungsindex des dahinter befindlichen Medium unverändert.

Fig. 8 zeigt eine Erfindungsgemäße Intraokularlinse (80) mit rein refraktiver Vorderfläche (81) und diffraktiver Rückfläche. Die makroskopische Form der Rückfläche, dargestellt durch die einhüllende gestrichelte Kurve (83) ist konvex, während die diffraktiv wirkenden Teilflächen (82, 83, 84) eine konkave Form aufweisen. Die Krümmungen der diffraktiven Teilflächen (82, 83, 84) entsprechen den Krümmungen der Wellenfronten (86) am jeweiligen Ort der Teilflächen, also am Übergang von der Linse zum Glaskörpermedium. Eine brechende Wirkung wird daher wiederum nur von der Frontfläche (81) erzeugt.

Zwischen benachbarten diffraktiven Teilflächen (82, 83, 84) oder Zonen sind Stufen erkennbar. Die geometrischen Höhen der Stufen sind so gewählt, dass die optische Weglängendifferenz (89) zwischen zwei diffraktiven Teilflächen (82, 83, 84) ein ganzzahliges Vielfaches N der Designwellenlänge ist. Von einer Teilfläche zur nächsten können dabei unterschiedliche Stufenhöhen realisiert werden, die Weglängenunterschieden verschiedener ganzzahliger Vielfacher der Designwellenlänge entsprechen. Auch können Stufen in verschiedenen Richtungen also mit unterschiedlichem Vorzeichen vorgesehen sein.

Bei einem ganzzahligen Vielfachen von vorzugsweise N>10 und einem Brechungsindex der Linse von 1.46 und einem Brechungsindex des Glaskörpers oder Kammerwasser 1.336 ergibt sich aus der Grundgleichung

$$h = N \cdot \frac{\lambda}{n_{Linse} - n_{umgebendesMedium}}$$

beispielsweise eine Höhe von ca. $N{\cdot}8{\cdot}\lambda$, wobei $N$ eine ganze Zahl $n_{Linse}$ und $n_{umgebendesMedium}$ die Brechungsindices und $\lambda$ die Designwellenlänge sind. Da sich für Medien mit unterschiedlichen Brechungsindizes im Glaskörperraum bei gleichem $N$ allerdings unterschiedliche Werte für $h$ ergeben, wird die Höhe h bevorzugt so gewählt, dass sie dennoch für beide Medien ein jeweils unterschiedliches ganzzahliges Vielfache N bzw. M des Weglängenunterschieds darstellt. Die geometrischen Höhen der Stufen h ergeben sich demnach aus

$$h = \frac{N \cdot \lambda}{n_{Linse} - n_{umgebendesMedium\_1}} = \frac{M \cdot \lambda}{n_{Linse} - n_{umgebendesMedium\_2}}.$$

wobei $N$ und $M$ ganze Zahlen sind und $n_{Linse}$, $n_{umgebendeMedium\_1}$ bzw. $n_{umgebendeMedium\_2}$ die Brechungsindices der Intraokularlinse und zweier unterschiedlicher bestimmter Tamponaden im Glaskörperraum des Auges sind.

Die Kurvenradien der einzelnen Teilflächen ändern sich erfindungsgemäß mit dem Abstand der Teilflächen zum Fokuspunkt 88.

Die Kanten der Stufen zwischen zwei benachbarten diffraktiven Teilflächen treffen vorzugsweise unter einem rechten Winkel auf die Teilflächen, verlaufen also in Strahlrichtung, um Streulicht an den Kanten zu reduzieren.

Insgesamt lassen sich auf diese Weise dünne Intraokularlinsen mit unterschiedlicher Brechkraft erzeugen, die alle eine Rückfläche mit optisch wirksamer Krümmung aufweisen, die der Krümmung der von der jeweilgen Vorderfläche einfallenden Wellenfront entspricht. Grundsätzlich kann dabei von Linsendesign zu Linsendesign sowohl die Dicke der Linse, die Verhältnisse des zentralen Linsenbereichs zu dem ringförmigen Linsenbereich als auch die Anzahl und Höhe der ringförmigen Zonen kann, u. a. in Abhängigkeit von dem Brechungsindex des Linsenmaterials, je nach gewünschter Brechkraft der Linse variiert werden. Auch kann die sägezahnförmige Ringstruktur zusätzlich oder ausschließlich an der Rückfläche der Intraokularlinse vorgesehen sein. Ebenfalls kann für den zentralen Linsenbereich und den ringförmigen Linsenbereich oder auch die einzelnen ringförmigen Zonen ein jeweils unterschiedliches Linsenmaterial mit unterschiedlichen Brechungsindizes verwendet werden, so dass sich noch weitere Variationsmöglichkeiten des Linsendesigns ergeben.

**Patentansprüche**

1. Intraokularlinse mit einem optischen Linsenteil, der eine der Retina zugewandte Rückfläche (12, 22, 42, 52, 62) aufweist, wobei wenigstens eine optisch wirksame Teilfläche der Rückfläche einen konkaven, zumindest näherungsweise sphärischen Krümmungsverlauf aufweist, dessen Krümmungsradius dem Abstand der Teilfläche der Rückfläche an der implantierten Intraokularlinse zur Retina auf der optischen Zentrumsachse entspricht und der optische Linsenteil ausgebildet ist, einfallende Lichtstrahlen ohne Brechung an der Rückfläche des optischen Linsenteils auf die Retina zu fokussieren.

2.  Intraokularlinse nach Anspruch 1,
    **dadurch gekennzeichnet, dass** der Krümmungsradius der rückwärtigen Teilfläche zwischen 12 mm und 30 mm insbesondere zwischen 13 mm und 23 mm liegt.

3.  Intraokularlinse nach einem der vorstehenden Ansprüche,
    **dadurch gekennzeichnet, dass** eine erste optisch wirksame Teilfläche mit zumindest näherungsweise sphärischem Krümmungsverlauf in einem zentralen, rein refraktiven Linsenbereich (25, 45, 55, 65) der Rückfläche ausgebildet ist.

4.  Intraokularlinse nach Anspruch 3,
    **dadurch gekennzeichnet, dass** wenigstens eine zweite optisch wirksame Teilfläche mit zumindest näherungsweise sphärischem Krümmungsverlauf in einem den zentralen Linsenbereich (25, 45, 55, 65) umgebenden diffraktiven Linsenbereich (26, 46, 56, 66) der Rückfläche konzentrisch an der ersten optisch wirksamen Teilfläche angrenzend ausgebildet ist, wobei der Krümmungsradius der zweiten optisch wirksamen Teilfläche deren Abstand zur Retina im Bereich größter Sehschärfe entspricht.

5.  Intraokularlinse nach Anspruch 3 oder 4,
    **dadurch gekennzeichnet, dass** weitere optisch wirksame Teilflächen mit zumindest näherungsweise sphärischem Krümmungsverlauf in dem ringförmigen diffraktiven Linsenbereich (26, 46, 56, 66) konzentrisch an der zweiten optisch wirksamen Teilfläche und des weiteren aneinander angrenzend ausgebildet sind, wobei der Krümmungsradius der weiteren optisch wirksamen Teilflächen jeweils deren Abstand zur Retina im Bereich größter Sehschärfe entspricht.

6.  Intraokularlinse nach Anspruch 5,
    **dadurch gekennzeichnet, dass** der Weglängenunterschied des Strahlenganges im Übergang von jeweils benachbarten optisch wirksamen Teilflächen ein ganzzahliges Vielfaches der Designwellenlänge ist.

7.  Intraokularlinse nach einem der Ansprüche 3 bis 9,
    **dadurch gekennzeichnet, dass** der zentrale Linsenbereich (25, 45, 55, 65) einen Durchmesser von etwa 4 mm aufweist.

8.  Intraokularlinse nach einem der Ansprüche 3 bis 6,
    **dadurch gekennzeichnet, dass** sich über die Vorderfläche zumindest abschnittsweise eine diffraktive Feinstruktur (31) zur Bildung einer multifokalen Linse erstreckt.


**Claims**

1.  An intraocular lens comprising an optical lens portion which has a rear face (12, 22, 42, 52, 62) facing the retina, wherein at least one optically active sub-area of the rear face has a concave, at least approximately spherical curvature, the radius of which corresponds to the distance of the sub-area of the rear face on the implanted intraocular lens from the retina along the centre optical axis, and the optical lens portion is configured to focus incident rays of light onto the retina without refraction at the rear face of the optical lens portion.

2.  The intraocular lens according to claim 1,
    **characterised in that** the radius of curvature of the rearward sub-area is between 12 mm and 30 mm, in particular between 13 mm and 23 mm.

3.  The intraocular lens according to any one of the preceding claims,
    **characterised in that** a first optically active sub-area having an at least approximately spherical curvature is formed in a central, purely refractive lens region (25, 45, 55, 65) of the rear face.

**4.** The intraocular lens according to claim 3,
**characterised in that** at least one second optically active sub-area having an at least approximately spherical curvature is formed in a diffractive lens region (26, 46, 56, 66) surrounding the central lens region (25, 45, 55, 65) of the rear face and concentrically adjoining the first optically active sub-area, wherein the radius of curvature of the second optically active sub-area corresponds to the distance thereof from the retina in the region of greatest visual acuity.

**5.** The intraocular lens according to claim 3 or 4,
**characterised in that** further optically active sub-areas having at least approximately spherical curvature are formed in the annular diffractive lens region (26, 46, 56, 66) concentrically adjoining the second optically active sub-area and also one another, wherein the radius of curvature of the further optically active sub-areas corresponds to their respective distance from the retina in the region of greatest visual acuity.

**6.** The intraocular lens according to claim 5,
**characterised in that** the difference in path length of the beam path at the transition from respective adjoining optically active sub-areas is an integer multiple of the design wavelength.

**7.** The intraocular lens according to any one of claims 3 to 9,
**characterised in that** the diameter of the central lens region (25, 45, 55, 65) is about 4 mm.

**8.** The intraocular lens according to any one of claims 3 to 6,
**characterised in that** a diffractive microstructure (31) extends over the front face, at least in sections thereof, to form a multifocal lens.

**Revendications**

**1.** Lentille intra-oculaire comprenant une partie de lentille optique, qui a une surface (12, 22, 42, 52, 62) arrière tournée vers la rétine, au moins une surface partielle, efficace optiquement de la surface arrière ayant un tracé de courbure concave au moins à peu près sphérique, dont le rayon de courbure correspond, sur l'axe du centre optique, à la distance de la surface partielle de la surface arrière de la lentille intra-oculaire implantée à la rétine et la partie de lentille optique est constituée pour focaliser sur la rétine un faisceau lumineux incident sans réfraction sur la surface arrière de la partie de lentille optique.

**2.** Lentille intra-oculaire suivant la revendication 1,
**caractérisée en ce que** le rayon de courbure de la surface partielle arrière est compris entre 12 mm et 30 mm et notamment entre 13 mm et 23 mm.

**3.** Lentille intra-oculaire suivant l'une des revendications précédentes,
**caractérisée en ce qu'**une première surface partielle efficace optiquement, ayant un tracé de courbure au moins à peu près sphérique, est constituée dans une partie (25, 45, 55, 65) de la lentille centrale purement réfringente de la surface arrière.

**4.** Lentille intra-oculaire suivant la revendication 3,
**caractérisée en ce qu'**au moins une deuxième surface partielle efficace optiquement, ayant un tracé de courbure au moins à peu près sphérique, est constituée dans une partie (26, 46, 56, 66) diffractive et entourant la partie (25, 45, 55, 65) centrale de la lentille de la surface arrière concentriquement par rapport à la première surface partielle efficace optiquement, le rayon de courbure de la deuxième surface partielle efficace optiquement correspondant à la distance à la rétine dans la partie de plus grande acuité visuelle.

**5.** Lentille intra-oculaire suivant la revendication 3 ou 4,
**caractérisée en ce que** d'autres surfaces partielles efficaces optiquement, ayant un tracé de courbure au moins à peu près sphérique, sont constituées dans la partie (26, 46, 56, 66) de lentille annulaire diffractive concentriquement à la deuxième surface partielle efficace optiquement et, en outre, en étant voisines les unes des autres, le rayon de courbure des autres surfaces partielles efficaces optiquement correspondant respectivement à leur distance à la rétine dans la partie de plus grande acuité visuelle.

**6.** Lentille intra-oculaire suivant la revendication 5,

**caractérisée en ce que** la différence de longueur de chemin de la marche des rayons dans la transition de surfaces partielles efficaces optiquement respectivement voisines est un multiple en nombre entier de la longueur d'onde de conception.

7. Lentille intra-oculaire suivant l'une des revendications 3 à 6,
**caractérisée en ce que** la partie (25, 45, 55, 65) de lentille centrale a un diamètre d'à peu près 4 mm.

8. Lentille intra-oculaire suivant l'une des revendications 3 à 6,
**caractérisée en ce que**, au moins par endroit, une structure (31) fine de diffraction s'étend, pour la formation d'une lentille multifocale, sur la surface avant.

Fig. 1

Fig. 2

Fig. 3

Fig. 4

Fig. 5

Fig. 6

Fig. 7

Fig. 8

**EP 1 863 411 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1185220 B1

- WO 03007849 A